# EUROPÄISCHE PATENTSCHRIFT

(11) **EP 1 273 577 B1**
(45) Veröffentlichungstag und Bekanntmachung des Hinweises auf die Patenterteilung: **20.12.2006**
(21) Anmeldenummer: 02011791.7
(22) Anmeldetag: 28.05.2002
(51) Int. Cl.: C07D 307/89

(54) **Verfahren zur Erzeugung von o-Xylol-Luft-Gemischen für die Phthalsäureanhydrid-Herstellung**
Process for the preparation of o-xylene-naphthalene-air mixtures for the production of phthalic anhydride
Procédé pour la préparation de mélanges de o-xylène-naphthalène-air pour la production d'anhydride phthalique

(30) Priorität: 05.07.2001 DE 10132627
(43) Veröffentlichungstag der Anmeldung: 08.01.2003
(73) Patentinhaber: Lurgi AG, 60295 Frankfurt am Main (DE)
(72) Erfinder: Domes, Helmuth, 63179 Obertshausen (DE); Gutermuth, Thomas, 63477 Maintal (DE); Feisel, Herbert, 65719 Hofheim (DE); Urban, Lutz, 61389 Schmitten (DE)

(56) Entgegenhaltungen:
- EP-A- 0 483 645
- DE-A- 2 839 831
- DE-A- 3 431 028
- US-A- 4 261 899
- US-A- 4 855 458
- DATABASE WPI Section Ch, Week 199203 Derwent Publications Ltd., London, GB; Class A41, AN 1992-022154 XP002211119 & SU 1 625 875 A (LYUBARSKII A G), 7. Februar 1991 (1991-02-07)

## Beschreibung

Die Erfindung betrifft ein Verfahren zur Erzeugung eines homogenen Gasgemisches (Feedgas) aus ortho-Xylol (o-Xylol) und Luft, das die Basis zur Herstellung von Phthalsäureanhydrid (PSA) durch Gasphasenoxidation im sogenannten PSA-Prozeß ist.

Die Feedgas-Erzeugung für den mit o-Xylol als Einsatzprodukt betriebenen PSA-Prozeß wird bislang wie folgt durchgeführt:
Die Prozeßluft wird mit Hilfe eines Gebläses aus der Umgebung angesaugt, filtriert, und anschließend auf ein Druckniveau verdichtet, das die Förderung des Luftstroms durch den Prozeßgas-Strang der PSA-Anlage erlaubt. Dieser Prozeßluftstrom wird in einem dem Gebläse nachgeschalteten Wärmetauscher in seinem Temperatumiveau angehoben. Parallel dazu wird flüssiges o-Xylol aus einem Vorratstank mit Hilfe einer Pumpe auf einen bestimmten Vordruck gebracht und durch einen Vorwärmer gefördert. In einem sogenannten Verdampfer wird das vorgewärmte o-Xylol parallel zur Luftströmung mit Hilfe eines Düsensystems in flüssiger Form in den Luftstrom eingesprüht. Die feinen o-Xylol-Tropfen verdunsten im Luftstrom vollständig, und schließlich wird durch eine Homogenisierungs-Stufe (z.B. einem statischen Mischer) eine Vergleichmäßigung der Konzentrations- und Temperaturverteilung im Gasstrom erreicht. Dieses Feedgas tritt anschließend in den mit Katalysator gefüllten Röhrenreaktor ein, wo eine partielle Oxidation von o-Xylol und Luftsauerstoff zu Phthalsäureanhydrid stattfindet.

Das oben beschriebene Verfahrensprinzip zur Feedgaserzeugung hat sich über Jahrzehnte im PSA-Prozeß bewährt, jedoch mit der sukzessiven Einführung von höheren o-Xylol-Beladungen im Luftstrom (> 80 g o-Xylol pro Nm³ Luft) auch potentielle Schwächen im Hinblick auf die Explosionssicherheit des Feedgasteils der PSA-Anlage gezeigt, die im folgenden dargelegt werden.
Die untere Explosionsgrenze eines gasförmigen Gemisches aus o-Xylol und Luft liegt bei 44 g/Nm³. Man hat festgestellt, daß die minimal erforderliche Energie zur Zündung des Gemisches mit zunehmender o-Xylol-Beladung stark herabgesetzt wird und daher eine erhöhte Sensibilität im Hinblick auf die Möglichkeit einer Explosion gegeben ist. Die Wirtschaftlichkeit des PSA-Verfahrens hängt in hohem Maße von der Beladung an o-Xylol pro Nm³ Luft ab. Grundsätzlich besteht die Forderungen, Anlagen mit einer Kapazität von 80 g o-Xylol/Nm³ Luft bis 120 g o-Xylol/Nm³ sicher zu betreiben.
Bezüglich der möglichen Ursachen einer Zündung im Feedgasteil einer PSA-Anlage speziell bei Einsatz des oben beschriebenen Verfahrens zur Feedgaserzeugung ist folgendes festzuhalten:
- Bei der Zerstäubung des flüssigen o-Xylols in den Sprühdüsen kommt es zum Aufbau statischer Elektrizität im o-Xylol-Tropfen selbst, deren Entladung über Einbauten im Verdampfer zu einem Funken führen kann, der wiederum Ursache einer Explosion sein kann.
- Die Ablagerung von Abdampfrückständen aus dem verdunsteten o-Xylol an Inneneinbauten des Verdampfers kann zum Aufbau von Feststoffschichten führen, die durch chemische Reaktion mit Sauerstoff aus der Prozeßluft pyrophore Substanzen bilden können, die wiederum potentielle Zündquellen für eine Explosion darstellen.

In der DE-A-2839831 wird ein Verfahren zur Herstellung eines Gasgemisches für die Naphthalin-Oxidation beschrieben, bei dem das Naphthalin in Abwesenheit von Sauerstoff verdampft, das verdampfte Naphthalin mit Sauerstoff gemischt und das Mischungsverhältnis von Naphthalin zum Sauerstoff durch Messung der Strömungsgeschwindigkeit geregelt wird. Bei diesem Verfahren unter Verwendung von Naphthalin wird eine Kapazität von 40 g Naphthalin /Nm³ Luft erreicht.
In der EP-B-0483645 wird ein Verfahren zur Erzeugung eines Feedgas-Gemisches aus Naphthalin, o-Xylol und Luft beschrieben. Hierbei wird o-Xylol zunächst unter Sauerstoffabschluß verdampft und anschließend durch flüssiges Naphthalin geleitet, damit sich die o-Xylol-Dämpfe mit Naphthalin aufsättigen. Dieses Gemisch wird dann der Prozeßluft zugeführt. Hierbei wird ein Kontakt des zu verdampfenden Naphthalins mit Luftsauerstoff vermieden, was sonst zur kontinuierlichen Bildung von teerartigen Nebenprodukten im Naphthalinverdampfer führen würde und eine gehäufte Notwendigkeit zu dessen Reinigung zur Folge hätte. Bei diesem Verfahren ist die Kapazität auf 100 g Kohlenwasserstoffe / Nm³ Luft begrenzt.

Der Erfindung liegt die Aufgabe zugrunde, ein Verfahren zur Herstellung o-Xylol-Luft-Gemische für die Phthalsäureanhydrid-Herstellung zu schaffen, mit dem eine Beladung von 80 g o-Xylol pro Nm³ Luft bis 120 g o-Xylol pro Nm³ Luft zu erreichen ist.
Erfindungsgemäß wird die Aufgabe dadurch gelöst, dass o-Xylol vollständig unter Ausschluss von Sauerstoff verdampft, danach überhitzt und dann mit sauerstoffhaltiger Luft vermischt wird und dieses Gemisch dem PSA-Reaktor zugeführt wird.
Um eine homogene Vormischung der beiden Komponenten zu bewirken, wird an der Stelle der Beimischung des o-Xylol-Dampfs zur Prozeßluft eine Gas-Gas-Mischeinrichtung vorgesehen, die als Spargersystem ausgeführt ist. Eine nachgeschaltete statische Mischeinrichtung stellt die vollständige Homogenisierung des Gemisches sicher.
Als Verdampfer kann ein Kettle-Type Verdampfer oder ein Naturumlauf-Verdampfer zum Einsatz kommen. Der Kettle-Type-Verdampfer stellt die technisch einfachere und damit kostengünstigere Lösung dar und ist daher im Regelfall vorzuziehen. Falls das o-Xylol hohe Gehalte an Nebenprodukten mit Styrol oder Cumol aufweist, kann es an den Verdampferflächen zu Ablagerungen kommen, die den Wärmeübergang behindern; dies läßt sich durch Einsatz eines Umlaufverdampfers vermeiden, da hier höhere Strömungsgeschwindigkeiten im Bereich der Verdampferflächen vorliegen.
Das o-Xylol muß bei einer Temperatur von mindestens 175°C verdampft werden, die einem Dampfdruck von 2,3 bar absolut entspricht. Damit wird sichergestellt, daß ein ausreichendes Druckgefälle zur Beimischung des o-Xylols zur Prozeßluft gegeben ist, die an der Zumischungsstelle des dampfförmigen o-Xylols einen Druck von ca. 1,5 bar absolut aufweist. Für die praktische Anlagenausführung wird für die o-Xylol-Verdampfung eine Temperatur von 180°C bis 205°C gewählt, was Dampfdrücken von 2,4 bar bis 4 bar absolut entspricht.
Eine wichtige Forderung an das Verdampfungssystem besteht darin, daß der Strom an o-Xylol-Dampf konstant bleibt, denn der Katalysator im PA-Reaktor kann bei Schwankungen der o-Xylol-Beladung im Luftstrom innerhalb kurzer Betriebszeiträume Schaden nehmen; dies trifft insbesondere bei hohen o-Xylol-Beladungen zu. Die Dampfzufuhr zum o-Xylol-Verdampfer schwankt normalerweise in gewissen Grenzen, was Schwankungen des zu verdampfenden o-Xylol-Stroms nach sich zieht. Dies kann durch Verwendung eines ausreichend dimensionierten Dampfspeichers für die Dampfzufuhr zum o-Xylol-Verdampfer zumindest weitgehend vermieden werden. Außerdem werden Schwankungen umso mehr gemindert, je höher der Druck im o-Xylol-Verdampfer ist.

Beispielhaft werden die Ausgestaltungsmöglichkeiten des Verfahrens mit Hilfe von Abbildungen erläutert.
Dabei zeigt
Fig. 1 ein Fließschema des Verfahrens mit einem Kettletype-Verdampfer,
Fig. 2 die o-Xylol-Verdampfung mit einem Natummlaufverdampfer
Fig 3 ein Spargerringsystem

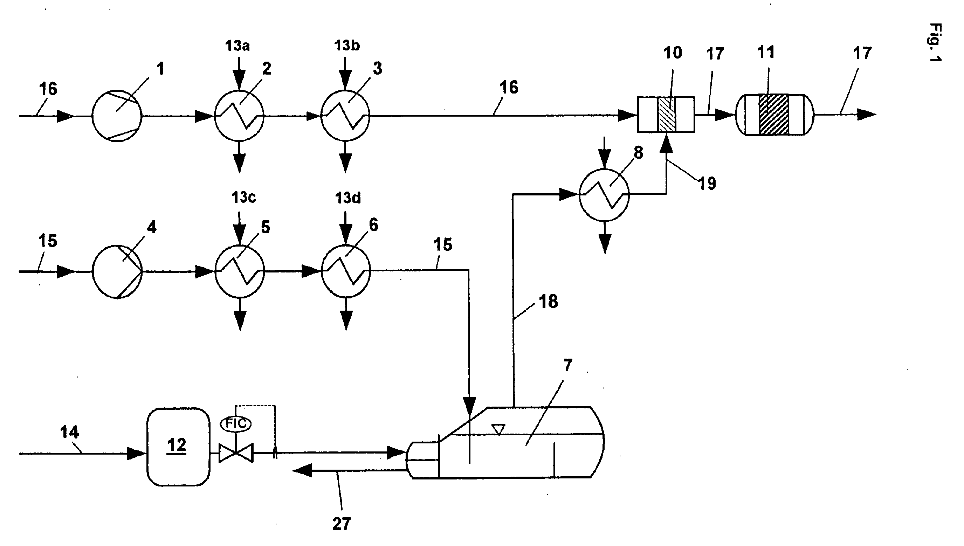

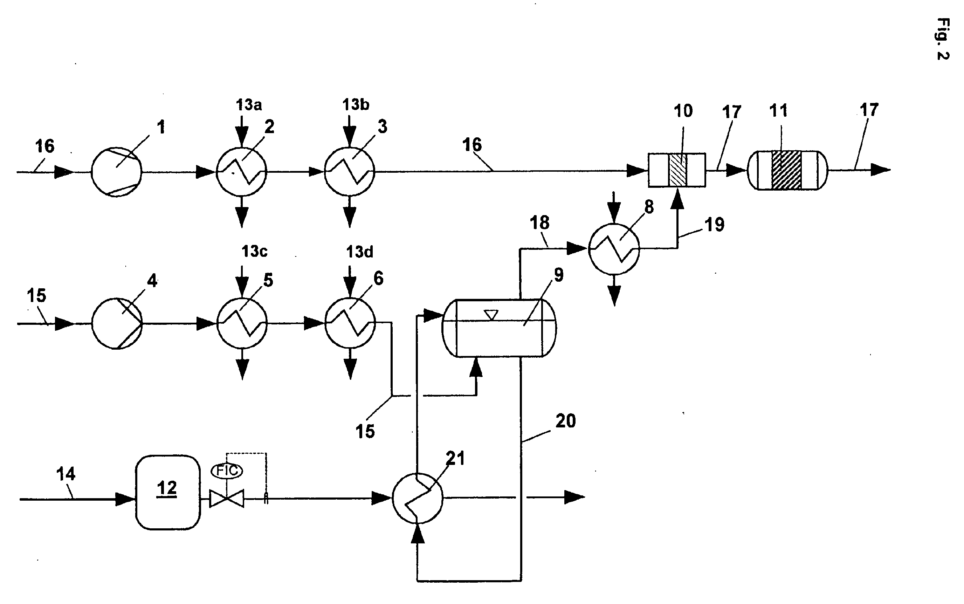

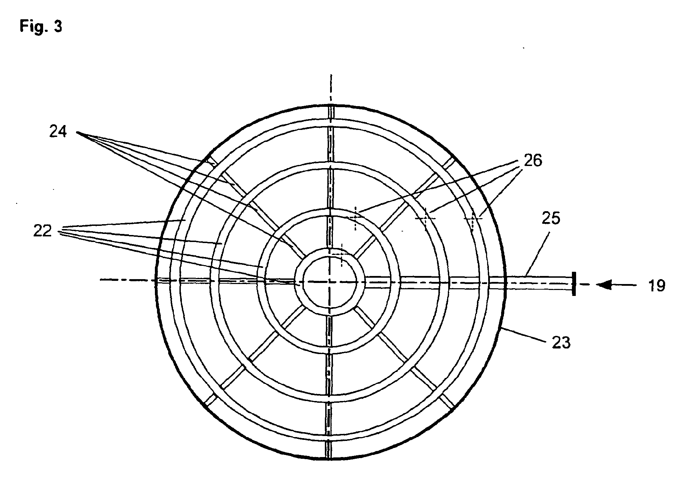

Nach Fig. 1 wird aus einem Vorratstank o-Xylol (15) mit Hilfe der o-Xylol-Pumpe (4) durch die beiden o-Xylol-Vorwärmer (5) und (6) gefördert, wo das o-Xylol mit Hilfe von Dampf (13c, 13d) auf 180°C erwärmt wird. Die o-Xylol-Pumpe (4) liefert dabei auf der Druckseite einen absoluten Druck von 2,5 bar. Anschließend gelangt das flüssige o-Xylol (15) in den Kettle-Type-Verdampfer (7). Dieser Kettle-Type-Verdampfer weist im Innern nicht dargestellte dampfbeheizte Rohrschlangen auf. Das o-Xylol (15) wird bei absoluten Drücken von 2,4 bar bis 4 bar verdampft, was einer Verdampfungstemperatur von 180°C bis 205°C entspricht. Als Heizmedium wird Sattdampf (14) zwischen 18 und 30 bar verwendet, der mit einer Temperatur von 210°C bei 18 bar bzw. 234°C bei 30 bar genügend Temperaturdifferenz zum zu verdampfenden Medium aufweist. Um den Sattdampfstrom zum o-Xylol-Verdampfer (7) konstant zu halten, kommt ein Dampfspeicher (12) zum Einsatz, der zwischen der Dampfzufuhr vom Dampfnetz und dem o-Xylol-Verdampfer (7) positioniert ist.
Der so erzeugte o-Xylol-Dampf (18) wird aus dem Dampfraum des Kettletype-Verdampfers (7) abgezogen und durch einen Überhitzer (8) geleitet, der den o-Xylol-Dampf (18) um 10°C überhitzt, um eine Kondensation des Dampfes zu vermeiden. Überschüssiges Kondensat (27) wird aus dem Verdampfer (7) abgezogen. Die Vermeidung der Kondensation von o-Xylol-Dampf ist für die Mengenregelung des o-Xylols von entscheidender Bedeutung, da diese über die Mengenmessung des o-Xylol-Dampfs erfolgt und es bei Kondensation von o-Xylol-Dampf zu Schwankungen in der o-Xylol-Beladung im Prozeßluftstrom kommen würde. Schließlich wird der überhitzte o-Xylol-Dampf (19) mit der Prozeßluft (16) in einer speziellen Mischeinrichtung (10) gemischt, die vom Prozeßluftgebläse (1) durch die zweistufig ausgeführten Vorwärmer (2) und (3) zum Reaktor hin gefördert wird. Die Mischeinrichtung (10) ist so konzipiert, daß an der Zumischstelle bereits eine gute Vermischung des überhitzten o-Xylols-Dampfes (19) mit der Prozeßluft (16) erreicht wird. Hierfür kommen Spargerringe zum Einsatz, die den o-Xylol-Dampf über den gesamten Rohrleitungsquerschnitt der Prozeßluftleitung verteilen. Die Prozeßluft (16) wird in den Vorwärmern (2, 3) auf ca. 150°C erwärmt. Der zusammengeführte Feedgasstrom (17) passiert einen statischen Mischer (11) zwecks Homogenisierung von Konzentration und Temperatur, was für den sicheren und effizienten Betrieb des Oxidationsreaktors in diesen hohen Beladungsbereichen von größter Bedeutung ist. Die möglichst homogene Vormischung des überhitzten o-Xylol-Dampfes (19) in der Mischeinrichtung (10) (siehe auch Fig. 3) ist erforderlich, da der statische Mischer (11) nur mit einer festliegenden Effizienz mischen kann und daher bereits ein gut homogenisiertes Gemisch an seinem Eintritt benötigt, um die erforderliche Mischungsgüte für den Reaktor gewährleisten zu können.
Anstelle der Verdampfung des o-Xylols in einem Kettle-Type-Verdampfer kann als Verdampfungsprinzip ein Naturumlauf eingesetzt werden, wie in Fig. 2 dargestellt:
Das o-Xylol (15) gelangt nach der Vorwärmung (5, 6) in die o-Xylol-Dampftrommel (9). Diese o-Xylol-Dampftrommel (9) wird von einem Naturumlauf gespeist.
Durch das Fallrohr (20) gelangt das siedende o-Xylol (15) in einen dampfbeheizten Verdampfer (21), der als Reboiler arbeitet. Dort verdampft ein Teil des durchströmenden o-Xylols bei absoluten Drücken von ca. 2,4 bis 4 bar und wird dann wieder durch den Naturumlauf in die o-Xylol-Dampftrommel (9) gefördert. Aus dem Dampfraum gelangen die Dämpfe in den Überhitzer (8). Hier wird der o-Xylol Dampf (18) um 10°C überhitzt, um eine Kondensation des Dampfes, wie zuvor beschrieben, zu vermeiden. Das weitere Verfahren ist identisch mit dem in Fig 1 beschriebenen Ablauf.
Fig. 3 zeigt ein Spargerringsystem, mit dem eine Vermischung des überhitzten o-Xylol-Dampfes (19) mit der Prozessluft erfolgt. Mehrere Ringe (22) sind konzentrisch zueinander innerhalb der Prozeßluft-Rohrleitung (23) angeordnet. Die Ringe (22) bestehen aus gebogenen und geschweißten Rohren. Der Abstand zwischen den Ringen (22) wird durch radiale Haltebleche (24) festgelegt. Über eine Rohrleitung (25) wird unter Druck überhitzter o-Xylol-Dampf (19) in alle Ringe geleitet. Die Ringe besitzen gleichmäßig über den Umfang verteilt eine Vielzahl von Austrittsbohrungen (26), über die der o-Xylol-Dampf austritt und sich dabei mit der Prozeßluft vermischt.

### Beispiel:

Für eine PSA-Anlage mit einer Jahreskapazität von 50000 Tonnen pro Jahr wird die o-Xylol-Verdampfung nach vorstehend genannten Prinzip durchgeführt, wobei die Anlage 8000 h im Jahr in Betrieb ist. Das o-Xylol weist eine Reinheit von 98 % auf. Da die Anlage bei einer Beladung von 100 g o-Xylol pro Nm³ Luft arbeitet und die Anlage einen Yield von 112 % (kg erzeugtes Rein PSA pro kg eingesetztes o-Xylol) erzielt, ist ein o-Xylol-Strom von 50000 to/a PSA x 1,12/8000 / 0.98 = 7143 kg o-Xylol / h erforderlich.
Der Luftstrom, den die Anlage zur Oxidation benötigt, beträgt 71430 Nm³/h.
Die o-Xylol-Pumpe fördert den o-Xylolstrom (20°C, atmosph. Druck), der am Pumpenaustritt einen Druck von 1,8 bar und eine Temperatur von 40°C aufweist. Im ersten mit Niederdruckdampf von 2,5 bar beheizten Wärmetauscher erreicht das o-Xylol eine Temperatur von 135°C. Im zweiten mit 18 bar beheizten Wärmetauscher wird der o-Xylol-Strom auf 175°C aufgeheizt. Beim Eintritt in den Verdampfer weist das o-Xylol einen Druck von 1,4 bar auf, wenn die Wärmetauscher und die Rohrleitungen einen Druckverlust von 0,4 bar aufweisen.
Die Dampfverbräuche der beiden Vorwärmer sind folgende:
1. Vorwärmer:
   Wärmestrom: 7143 kg / h / 3600 s/h x 2,1 kJ / kg K x (135°C - 40°C) = 396 kW
   Dampfbedarf: 396 kW / 2153 kJ / kg x 3600 s/h = 662 kg / h (2.5 bar)
2. Vorwärmer:
   Wärmestrom: 7143 kg / h / 3600 s/h x 2,1 kJ / kg K x (175°C - 135°C) = 167 kW
   Dampfbedarf: 167 kW / 1900 kJ / kg K x 3600 s/h = 316 kg / h (18 bar)
   Der Verdampfer muß in der Stunde 7143 kg o-Xylol verdampfen. Hierfür ist ein Wärmestrom von: 7143 kg/h / 3600 s/h x [315 KJ/kg + (180 °C - 175 °C) x 2,15 kJ/kg K] = 1365 kW
   erforderlich, was einer Heizdampfmenge (18 bar) von
   1365 kW / 1900 kJ/kg x 3600 s /h = 2586 kg/h bedarf.
Im Falle des Naturumlaufverdampfersystems zirkulieren
7143 kg / h / 0,15 = 47620 kg /h o-Xylol im Verdampferkreislauf.
Die aus der Dampftrommel austretenden o-Xylol Dämpfe werden im Überhitzer um 10 K erhitzt, so daß sie am Überhitzeraustritt eine Temperatur von 190 °C aufweisen.

Der Überhitzer benötigt dafür eine Wärmeleistung von
7143 kg/h / 3600 s/h x 1.9 kJ/kg K x (190°C - 180°C) = 38 kW
was einer Heizdampfmenge von 38 kW / 1900 kJ/kg x 3600 s/h = 72 kg/h (18 bar)
entspricht.
Die überhitzten o-Xylol-Dämpfe weisen am Austritt des Verdampfers einen Druck von 1,3 bar auf. Diese Dämpfe werden anschließend in den Prozeßluftstrom entspannt, dessen Druck an dieser Stelle ca. 0,5 bar beträgt.

## Patentansprüche

1. Verfahren zur Herstellung eines o-Xylol-Luft-Gemisches für die Phthalsäureanhydrid-Herstellung, **dadurch gekennzeichnet,**
**dass** o-Xylol vollständig unter Ausschluss von Sauerstoff verdampft, danach überhitzt und dann mit der Prozeßluft vermischt wird und dieses Gemisch dem PSA-Reaktor zugeführt wird.

2. Verfahren nach Anspruch 1, **dadurch gekennzeichnet, daß** der Verdampfer für das o-Xylol ein Naturumlauf-Verdampfer (9) oder ein Kettle-Type-Verdampfer (7) ist.

3. Verfahren nach Anspruch 1, **dadurch gekennzeichnet, daß** die Anlage eine Beladung von 80 g o-Xylol pro Nm³ Luft bis 120 g o-Xylol pro Nm³ Luft hat.

4. Verfahren nach Anspruch 1, **dadurch gekennzeichnet, daß** zur Heizdampfversorgung ein Dampfspeicher (12) dem o-Xylol-Verdampfer (7, 9) vorgeschaltet ist.

5. Verfahren nach Anspruch 1, **dadurch gekennzeichnet, daß** die Beimischung des o-Xylol-Dampfes mit der Prozessluft durch eine Mischeinrichtung erfolgt, die als Spargerringsystem ausgeführt ist.

## Claims

1. A process for producing an o-xylene-air mixture for the production of phthalic anhydride, **characterized in**
**that** o-xylene is completely evaporated by exclusion of oxygen, is then superheated and then mixed with the process air, and this mixture is supplied to the PA reactor.

2. The process as claimed in claim 1, **characterized in that** the evaporator for the o-xylene is a natural-circulation evaporator (9) or a kettle-type evaporator (7).

3. The process as claimed in claim 1, **characterized in that** the plant has a loading of 80 g of o-xylene per Nm³ of air to 120 g of o-xylene per Nm³ of air.

4. The process as claimed in claim 1, **characterized in that** for supply with heating steam a steam accumulator (12) is provided upstream of the o-xylene evaporator (7, 9).

5. The process as claimed in claim 1, **characterized in that** the admixture of the o-xylene vapor to the process air is effected by a mixing device designed as sparger ring system.

## Revendications

1. Procédé pour la préparation d'un mélange o-xylène-air pour la préparation d'anhydride de l'acide phtalique, **caractérisé**
**en ce qu'**on procède à une évaporation complète du o-xylène à l'abri de l'oxygène, avant de le soumettre à une surchauffe et avant de le mélanger avec l'air du processus et d'acheminer ce mélange au réacteur de préparation de l'anhydride de l'acide phtalique.

2. Procédé selon la revendication 1, **caractérisé en ce que** l'évaporateur pour le o-xylène est un évaporateur du type à circulation naturelle (9) ou un évaporateur du type à chaudière (7).

3. Procédé selon la revendication 1, **caractérisé en ce que** l'installation possède une charge de 80 g de o-xylène par Nm³ d'air à 120 g de o-xylène par Nm³ d'air.

4. Procédé selon la revendication 1, **caractérisé en ce que**, pour l'alimentation de vapeur de chauffage, un accumulateur de vapeur (12) est monté à l'avant de l'évaporateur du o-xylène (7, 9).

5. Procédé selon la revendication 1, **caractérisé en ce que** l'addition par mélange de la vapeur de o-xylène à l'air du processus a lieu via un mécanisme de mélange qui est réalisé sous la forme d'un système à collier d'aspersion.
